# EUROPEAN PATENT SPECIFICATION

(11) **EP 2 744 548 B1**
(45) Date of publication and mention of the grant of the patent: **08.07.2020**
(21) Application number: 12781462.2
(22) Date of filing: 01.10.2012
(51) Int. Cl.: A61M 16/06

(54) **ERGONOMICALLY FORMED HEADGEAR STRAPS**
ERGONOMISCH GEFORMTE KOPFBEDECKUNGSBÄNDER
SANGLES ERGONOMIQUES POUR HARNAIS

(30) Priority: 06.10.2011 US 201161543838 P
(43) Date of publication of application: 25.06.2014
(73) Proprietor: Koninklijke Philips N.V., 5656 AG Eindhoven (NL)
(72) Inventor: CHODKOWSKI, Lauren Patricia, NL-5656 AE Eindhoven (NL); HO, Peter Chi Fai, NL-5656 AE Eindhoven (NL)
(74) Representative: Philips Intellectual Property & Standards
(86) International application number: PCT/IB2012/055250
(87) International publication number: WO 2013/050920

(56) References cited:
- JP-A- 2005 102 818
- US-A- 4 468 815
- US-A1- 2009 250 065
- US-A1- 2011 197 341

## Description

### CROSS-REFERENCE TO RELATED APPLICATIONS

This patent application claims the priority benefit under 35 U.S.C. § 119(e) of U.S. Provisional Application No. 61/543,838 filed on October 6, 2011.

### BACKGROUND OF THE INVENTION

### 1. Field of the Invention

The present invention pertains to headgear for use in securing devices, such as respiratory masks, to a human head, and, more particularly, to ergonomically formed strap members for use in such headgear. The invention further pertains to a method of ergonomically forming selected portions of a headgear.

### 2. Description of the Related Art

There are numerous situations where it is necessary or desirable to deliver a flow of breathing gas non-invasively to the airway of a patient, i.e., without intubating the patient or surgically inserting a tracheal tube in the patient's esophagus. For example, it is known to ventilate a patient using a technique known as non-invasive ventilation. It is also known to deliver continuous positive airway pressure (CPAP) or variable airway pressure, which varies with the patient's respiratory cycle, to treat a medical disorder, such as sleep apnea syndrome, in particular, obstructive sleep apnea (OSA), or congestive heart failure.

Non-invasive ventilation and pressure support therapies involve the placement of a respiratory patient interface device including a mask component that is typically secured on the face of a patient by a headgear assembly. The mask component may be, without limitation, a nasal mask that covers the patient's nose, a nasal cushion having nasal prongs that are received within the patient's nares, a nasal/oral mask that covers the nose and mouth, or a full face mask that covers the patient's face. It is known to maintain such devices on the face of a wearer by a headgear having one or more straps adapted to fit over/around the patient's head. Because such respiratory patient interface devices are typically worn for an extended period of time, it is important for the headgear to maintain the mask component in a desired position while doing so in a manner that is comfortable to the patient.

US 2009/0250065 A1 informs about a method for manufacturing a headgear. The method comprises a cutting off step to cut off a member forming a closed curved belt portion from a planar material.

Conventional headgear assemblies are commonly formed of panels and/or straps formed from fabric that is die-cut from generally flat sheet materials. Without the use of rigid reinforcement in headgear, the fabric panels often deform undesirably and result in buckling and mismatching when placed on a patient's head. Some of these issues can be self-compensating by stretching due to the elasticity of the materials used; however, such stretching may cause undesirable pressure points and unwanted tensions, thus causing discomfort to the patient. Thus, there is still room for improvement in headgear and straps for use therein.

### SUMMARY OF THE INVENTION

The invention is defined by the appended claims. Accordingly, it is an object of the present invention to provide an improved strap member for use in a headgear for securing a patient interface device to the head of a patient that overcomes the shortcomings of conventional headgear. The present invention provides a means for relieving unwanted tension or alternately adding tension where needed. The stitches and/or sewn cuts also can alter the direction and amount of stretching through careful placement of the material. The stitches also provide local reinforcement where the amount of reinforcement varies depending on the type of stitching used. This technique of placing cuts and stitches has been demonstrated in the various example embodiments described herein.

This object is achieved according to one embodiment of the present invention by providing a method of geometrically forming a portion of a headgear to conform to a selected portion of an intended user's head. The method comprises determining a triangular region on the portion of the headgear that generally coincides with the selected portion of the intended user's head, the triangular region being defined by a first side, a second side and a third side. The method further comprises forming a curved and cupped region in the portion of the headgear by bringing together and coupling the second and third sides of the triangular region. Bringing together and coupling the second and third sides of the triangular region comprises folding the triangular region along a fold line disposed within the triangular region.

The first side may be disposed on an outer edge of the portion of the headgear and the second and third sides may extend into the portion of the headgear away from the first side.

The first side of the triangular region may be disposed within the portion of the headgear.

Determining a triangular region may further comprise determining a another triangular region adjacent the triangular region, the another triangular region being defined by another first side, another second side, and another third side. The another first side coinciding with the first side. Forming a curved and cupped region in the portion of the headgear may further comprise bringing together and coupling the another second side and the another third side of the another triangular region.

Coupling together the adjacent sides may comprise at least one of one of stitching or gluing the adjacent sides together.

### BRIEF DESCRIPTION OF THE DRAWINGS

FIG. 1 is an elevational view of a portion of strap being modified according to the present invention;
FIG. 2 is a view of the strap of FIG. 1 modified according to an embodiment the present invention oriented to show curvature of the strap;
FIG. 3 is another view of the strap of FIG. 1 modified according to an embodiment of the present invention oriented to show cupping of the strap;
FIG. 4 is an example of the stages involved in forming a curved and cupped portion according to an example of the present disclosure;
FIG. 5 is an example of the stages involved in forming a curved and cupped portion according to another example of the present disclosure;
FIG. 6 is an example of the stages involved in forming a curved and cupped portion according to yet another embodiment of the present invention;
FIG. 7A is a side view of headgear incorporating modified portions in accordance with an embodiment of the present invention;
FIG. 7B is a rear view of the headgear of FIG. 7A;
FIG. 7C is a sectional view a portion of the headgear of FIG. 7A taken along line C-C of FIG. 7A;
FIG. 7D is a detail view of the portion of the headgear of FIG. 7A indicated generally at D;
FIG. 8 is a side view of a headgear incorporating modified portions in accordance with an embodiment of the present invention;
FIG. 9 is a side view of a headgear incorporating modified portions in accordance with an embodiment of the present invention;
FIG. 10A is a side view of a headgear incorporating modified portions in accordance with an embodiment of the present invention;
FIG. 10B is a rear view of the headgear of FIG. 10A;
FIG. 11A is a side view of a headgear incorporating modified portions in accordance with an embodiment of the present invention;
FIG. 11B is a rear view of the headgear of FIG. 11A;
FIG. 12A is a side view of a headgear incorporating modified portions in accordance with an embodiment of the present invention;
FIG. 12B is a rear view of the headgear of FIG. 12A;
FIG. 13A is an upward view of a chinstrap incorporating modified portions in accordance with an embodiment of the present invention;
FIG. 13B is a view of a chinstrap that is modified to form the chinstrap of FIG. 13A;
FIG. 14 is a side view of a headgear incorporating modified portions in accordance with an embodiment of the present invention; and
FIG. 15 is a side view of a headgear incorporating modified portions in accordance with an embodiment of the present invention.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein. Like numbers refer to like elements throughout.

The present invention provides geometric forming of a strap or other portion of a headgear formed from a planar, or generally planar, sheet of material so that the selected portion is able to better conform to the shape of a user's head. Because the head has may contours, geometric forming is one way to relieve unwanted tension and/or add tension where it is needed. Geometric forming also provides a means for reducing and/or eliminating undesirable buckling or mismatching. As described in detail below, geometric forming can also alter the direction and amount of stretching through careful placement on the material.

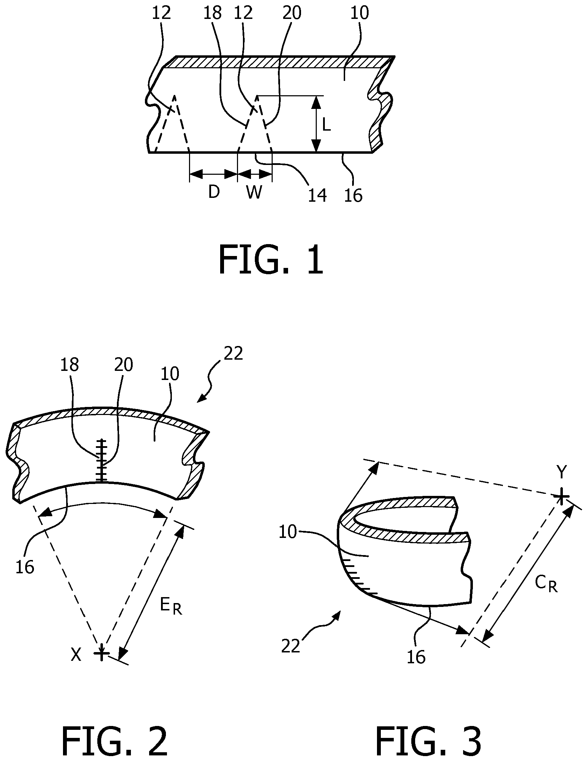

FIGS. 1-3 show an example of an embodiment of the present invention applied to a strap 10 initially formed from a generally planar fabric material such as used in headgear assemblies for use in securing a patient interface device to the head of a patient (user). Such strap 10 may be formed from laminated, woven, knit, or other suitable material and is preferably die cut to achieve the intended shape.

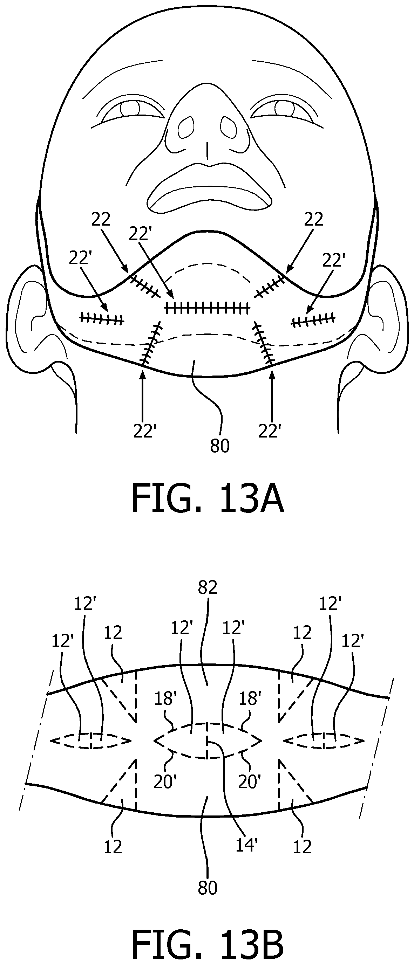

In order to geometrically form strap 10 to coincide with the shape of the selected portion of a user's head, the present invention utilizes strategically sized and placed triangular regions 12 in strap 10, such as shown in dashed lines in FIG. 1. Each triangular region 12 is formed or defined by a first side 14, a second side 18 and a third side 20. In the example embodiment illustrated in FIGS. 1-3, first side 14 is disposed on an outer edge 16 of strap 10 and second and third sides 18, 20 extend into strap 10 from first side 14 and outer edge 16. Preferably, second and third sides 18, 20 are of similar length and oriented oblique to outer edge 16. Although shown as being generally linear, it is to be appreciated that second and third sides 18, 20 may also be of a curved shape without varying from the scope of the present invention. It is also to be appreciated that in certain applications first side 14 may also be disposed on an internal portion of the strap, such as shown in the example illustrated in FIG. 13, as will be discussed below.

After a particular triangular region 12 has been determined, as discussed further below, the second and third sides 18, 20 of triangular region 12 are then brought together and coupled to form a region that is generally both curved about a first point X (see FIG. 2) and curled or cupped about a second point Y (see FIG. 3), where first and second points X and Y may or may not coincide depending on the layout of the particular embodiment. By coupling second and third sides 18, 20 of triangular region 12 together, side 16 of strap 10 is shortened, while maintaining the length of the other side (not numbered) of strap 10. When second and third sides 18, 20 of triangular region 12 are coupled together, the difference in the lengths of the sides of strap 10 is compensated for by outward curvature (see FIG. 2) and cupping (see FIG. 3) of a portion 22 of strap 10, hereinafter the "curved and cupped portion 22".

The radii and depth of the curving and cupping can be regulated through the length L and the width W of triangular region 12 (see FIG. 1). The length L and width W of each triangular region 12 are determined by the curvature seen at the anatomic area of interest on the user's head. Increasing the width W of triangular region 12 decreases the fabric edge radius E_{R} (see FIG. 2), while increasing the length L of the triangular region increases the cupping radius C_{R} (see FIG. 3). In regions where multiple triangular regions 12 are utilized, decreasing the distance D (FIG. 1) between adjacent triangular regions 12 also decreases the fabric edge radius E_{R} (conversely increasing the distance D increases the fabric edge radius E_{R}). As will be appreciated from the further discussion below, triangular regions 12 are selectively placed on headgear to create the same general shape or contour as the anatomic area of interest on the user's head.

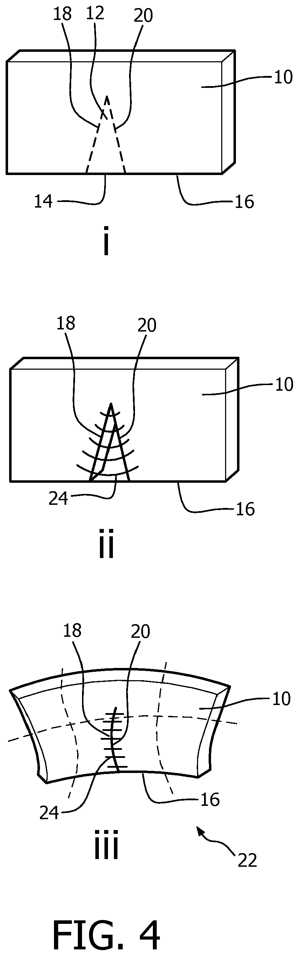

The coupling of second and third sides 18 and 20 of triangular region 12 may be carried out in a number of different manners. For example, referring to stages i-iii. of FIG. 4, triangular region 12 may be completely removed from strap 10 prior to coupling second and third sides 18, 20 by cutting strap 10 along second and third sides 18 and 20 of triangular region 12. After triangular region 12 has been removed from strap 10, second and third sides 18, 20 are then coupled together via stitching 24 or other suitable coupling means. Such method in which triangular region 12 is removed from strap 10 is generally preferred when strap 10 is formed from a relatively thick fabric material (e.g., without limitation, neoprene) so as to avoid undesirable thickening or bulking of strap 10 in or near curved and cupper portion 22.

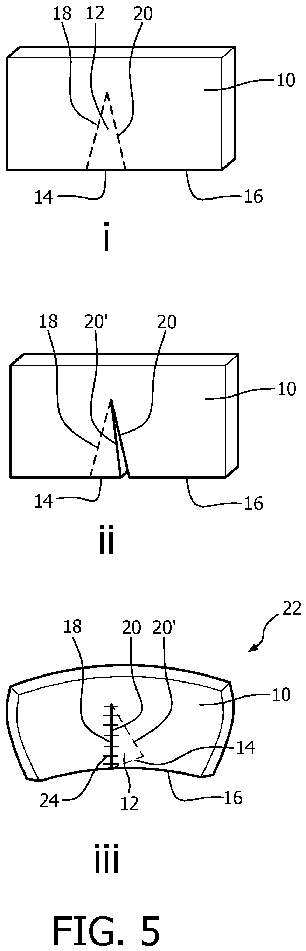

As another example, such as shown in stages i-iii. of FIG. 5, strap 10 may be cut along only one of second and third sides 18, 20 (in the illustrated example of FIG. 5, side 20 has been cut forming edges 20 and 20') while leaving the other of second and third sides 18, 20 (second side 18 in the example of FIG. 5) integral with strap 10. In such an embodiment triangular region 12 can then be tucked behind (or otherwise suitable folded with respect to) the portion of strap 10 disposed adjacent triangular region 12 when second and third sides 18, 20 are coupled. In such embodiment, second and third sides 18, 20 may be readily coupled via stitching 24, glue (preferably between tucked triangular region 12 and the corresponding portion of the backside of strap 10), a combination of stitching and glue, or any other suitable means.

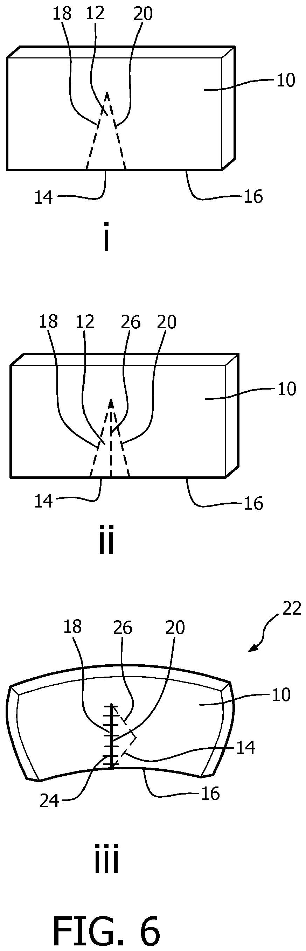

As yet another example, such as shown in stages i-iii. of FIG. 6, second and third sides 18, 20 of triangular region 12 may be brought together and subsequently coupled by folding triangular region 12 into one or more pleats by folding along one or more fold lines 26, thus requiring no cutting whatsoever of strap 10. In such embodiment, second and third sides 18, 20 may be readily coupled via stitching 24, glue (preferably applied to the faces of triangular portion 12 separated by fold line 26, a combination of stitching and glue, or any other suitable means.

Having thus described the basic concepts of the invention, application of such concepts in a number of example applications will now be provided. It is to be appreciated that shaping of the headgear fabric without the use of rigid stiffeners or multiple materials as described herein can be applied to a number of headgear applications to improve fit, comfort, and efficacy. The techniques can be used to make any soft headgear more ergonomic. By adding such shaping or contouring to the fabric, a more ergonomic fit may be seen for example, without limitation, around the cheek bone, the base of the skull/occipital area, the crown of the head, chin, and temporal/parietal curve around the ear.

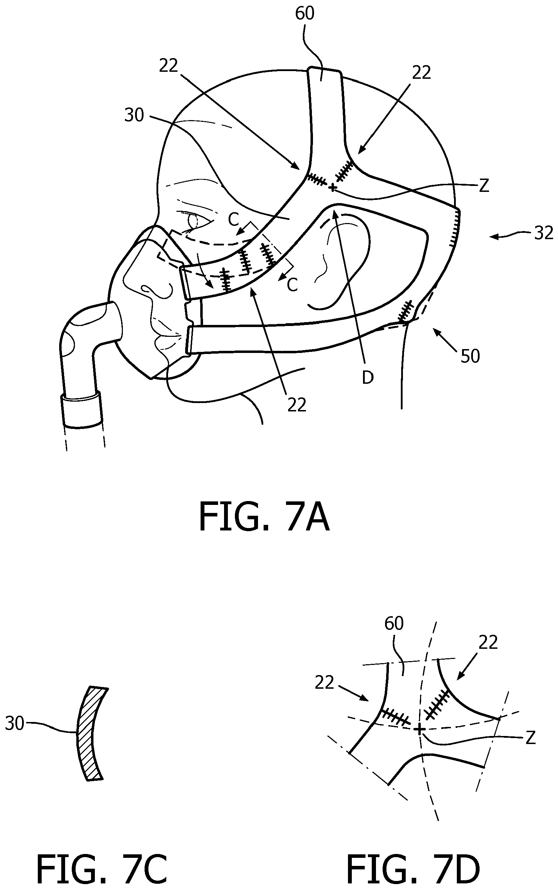

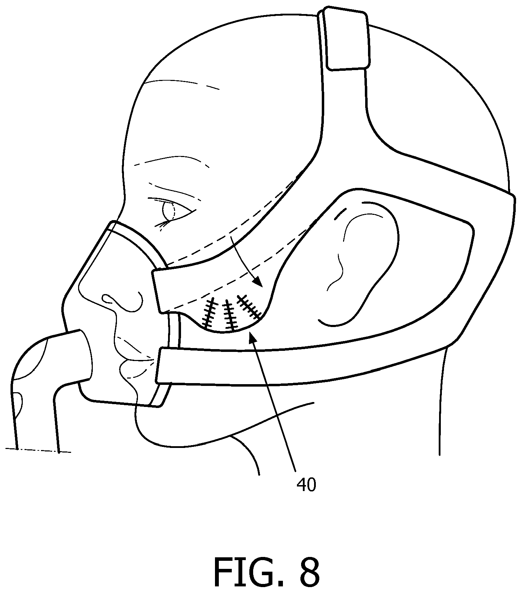

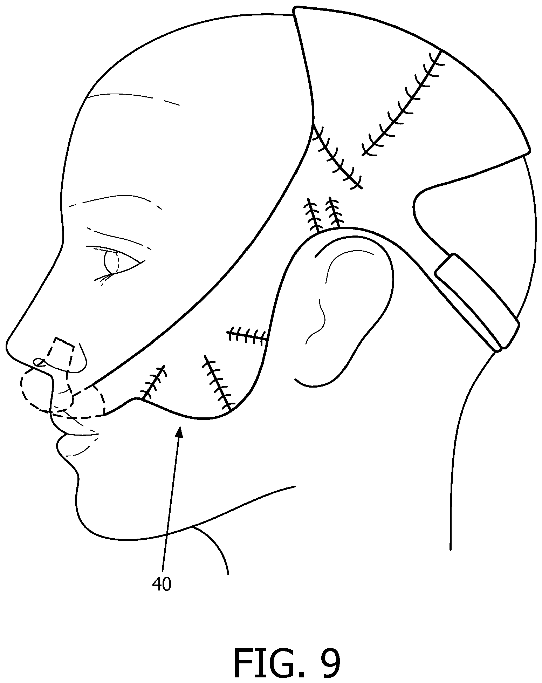

For example, accommodating for the cheek bone curvature can be accomplished in several ways. As shown in the example of FIGS. 7A-7D, providing alternating curved and cupped portions 22 on a strap material 30 of headgear 32 creates a chute-like shape (FIG. 7C) along the cheekbone of a patient. A chute-like strap around the cheek bone provides better support by hugging the zygomatic arch as well as directing forces from the mask to the headgear in a more effective manner. By preloading the semi-elastic strap on certain sides, the force vector created by the mask can be distributed evenly, especially in the event of a curved strap. Hugging the zygomatic arch can also be accomplished by using the techniques described herein to make a cheek cuff 40, such as shown in the example embodiments of FIGS. 8 and 9. By utilizing such techniques, the unique characteristics of the cheek bone are utilized to keep the headgear secure while maintaining an adequate eye clearance with the strapping.

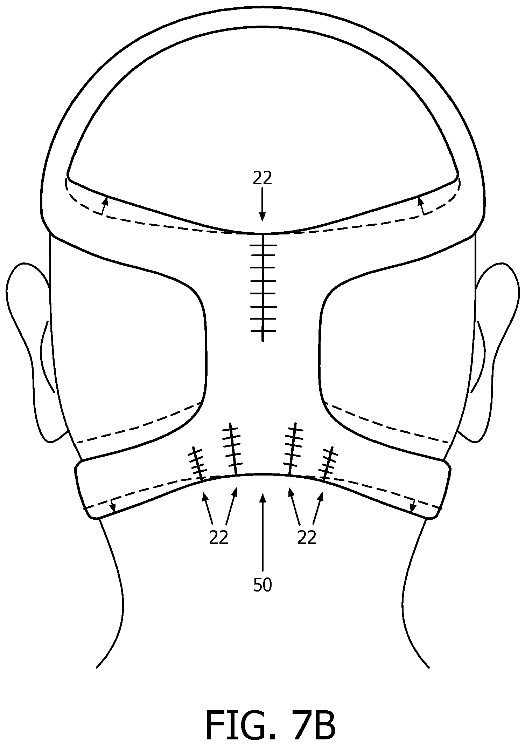

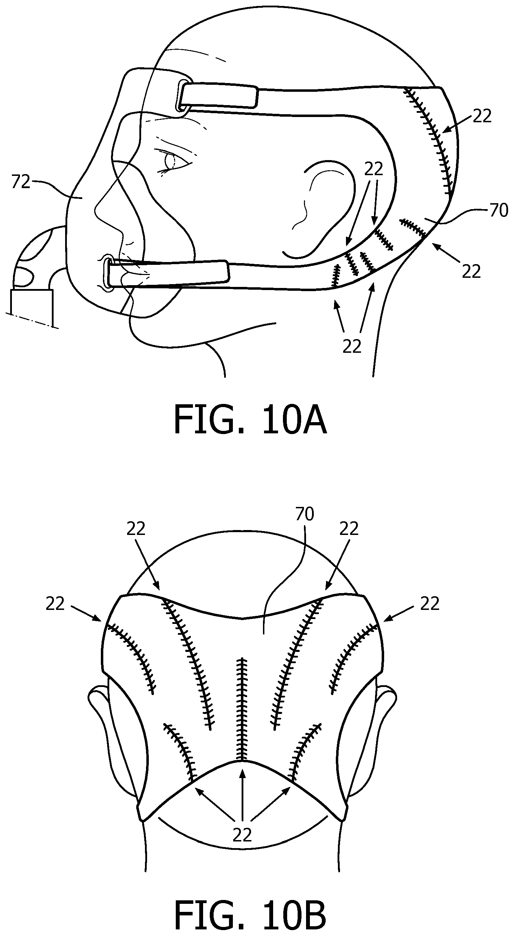

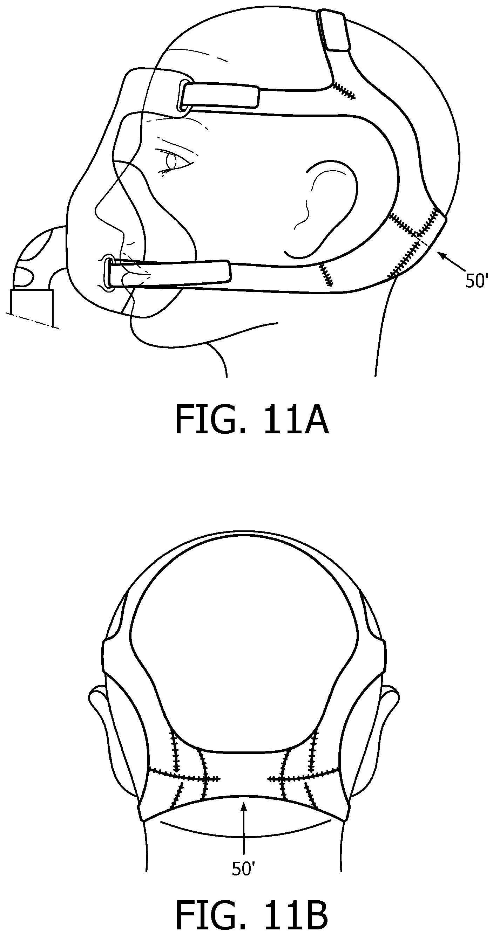

As another example, the base of the skull is a very contoured feature of the head for which a soft, single material headgear does not accommodate. Utilizing the concepts of the present invention, a 'contoured panel' 50, such as shown in FIGS. 7A and 7B, can be made to achieve a better fit around the occipital region of the skull. Such contouring also provides for improved neck mobility by hugging the occipital area and bringing the material from which the back panel is formed off the neck. Other examples of embodiments that contour around this area are seen in FIGS. 10A-B and 11A-B.

As another example, as shown in FIG. 7A, the top strap 60 used for a number of headgear designs can have a lot of mobility when using a single material and decreases efficacy as well as comfort. Contouring the material around the base of strap 60 as well as decreasing the elasticity (thread is not elastic, therefore addition of a stitch oriented correctly will decrease the elasticity locally where the stitch is placed) provides stability to strap 60. As shown in FIGS. 7A and 7D, placing two curved and cupped portions 22 at an angle toward a center point Z creates a 'clam shell' (see FIG. 7D) shape which can be used to form the material to the head, as well as preload/redirect the elasticity of the material to get a tighter fit in the temporal region.

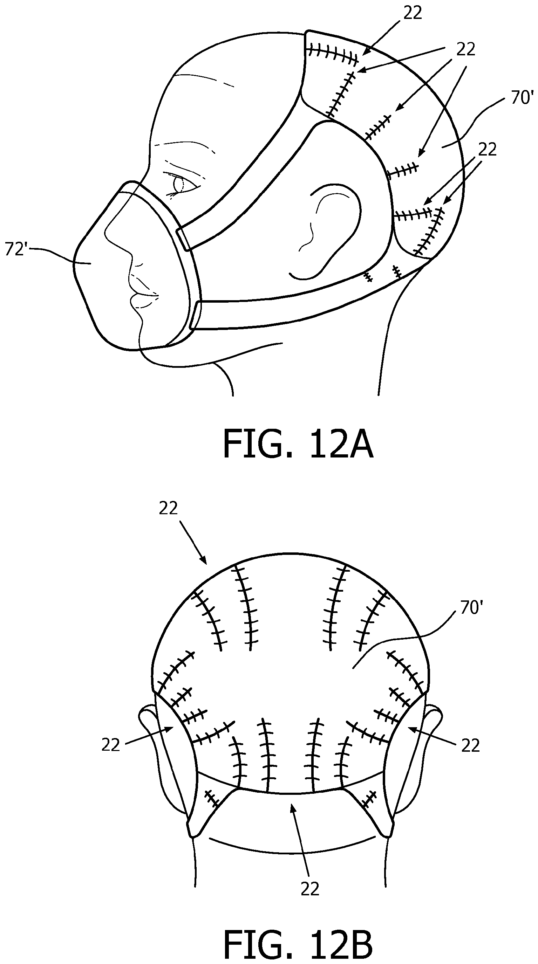

As another example, the concepts described herein can also be utilized with the contours of the crown of the head to make a more stable single material, soft headgear. The shape of the material can be matched to the occipital, parietal, and temporal regions to form a crown cap that provides excellent stability and eliminates the need for straps around the back of the head. Examples of such applications are shown in FIGS. 10A and 10B, as well as FIGS. 12A and 12B. Previously, straps were necessary for single material soft headgear to provide any contouring or stability. Straps are not as stable and often 'slip', causing a loss of functionality. The ability to contour a single panel, such as panels 70 and 70' to support a mask 72, 72' eliminates this problem.

Chin strap fittings, such as chin strap 80 of FIG. 13B, can also be enhanced according to the present invention by selecting and modifying triangular regions 12 and 12'. More particularly, a single piece of material 82 can be contoured to the chin of a user, such as shown in FIG. 13A by utilizing a number of curved and cupped portions 22 and 22' to fit the chin more closely. Curved and cupped portions 22 are formed using triangular regions 22 formed at edges (not numbered) of chin strap 80, while curved and cupped portions 22' are formed using triangular regions 12' (having first side 14', second side 18', and third side 20') formed within material 82 adjacent another triangular region 12'.

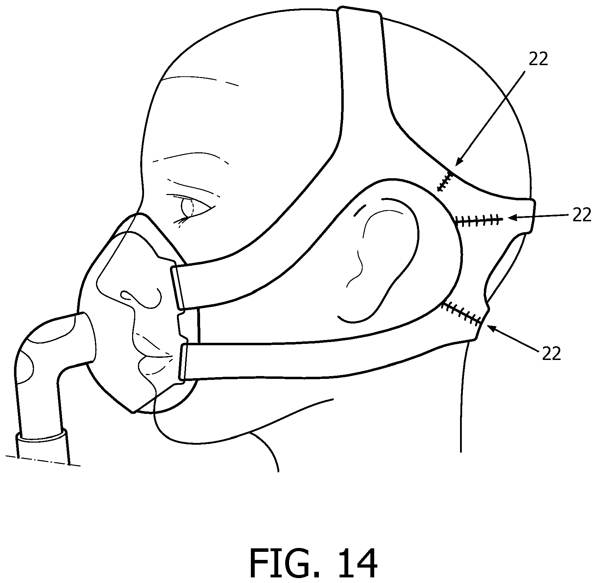

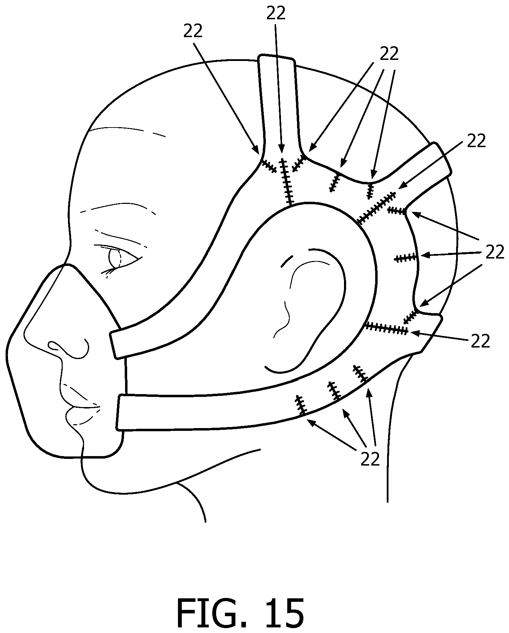

In conventional headgear designs that includes a C-shape around the ear, the use of a single material has commonly resulted in buckling and an inadequate distribution of forces. In an embodiment of such headgear that has been modified in accordance with the present invention, compensation for the local anatomy was made. To account for the curvature in the temporal and parietal regions, longer cuts provide a large cupping radius (FIG. 14). In FIG. 15, a C-shape headgear is also used; the straps are reinforced with stitching to establish less mobility and the longer cuts around the curve of the C are used to, again, compensate for the curvature in the parietal/temporal region.

It can be appreciated from the foregoing that the present invention provides improvements to headgear or portions thereof initially formed from planar or generally planar pieces of fabric. These improvements enhance the fit and thus the comfort of the headgear when worn by a user. In particular, the present invention provides for selected portions of a headgear to remain securely placed in desired locations, thus reducing undesirable unevenness in tensions throughout the headgear.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A method of geometrically forming a portion (10) of a headgear for securing a patient interface device to the head of a patient to conform to a selected portion of an intended user's head, the method comprising:
determining a triangular region (12, 12') on the portion of the headgear that generally coincides with the selected portion of the intended user's head, the triangular region being defined by a first side (14, 14'), a second side (18, 18') and a third side (20, 20'); and
forming a curved and cupped region (22, 22') in the portion of the headgear by bringing together and coupling the second and third sides of the triangular region,
**characterized in that** bringing together and coupling the second and third sides comprises folding the triangular region along a fold line (26) disposed within the triangular region.

2. The method of claim 1, wherein the first side is disposed on an outer edge (16) of the portion of the headgear and the second and third sides extend into the portion of the headgear away from the first side.

3. The method of claim 1, wherein the first side (14') of the triangular region (12') is disposed within the portion of the headgear.

4. The method of claim 3, wherein determining a triangular region further comprises determining a another triangular region (12') adjacent the triangular region (12'); wherein the another triangular region is defined by another first side (14'), another second side (18'), and another third side (20'), the another first side coinciding with the first side; and wherein forming a curved and cupped region in the portion of the headgear further comprises bringing together and coupling the another second side and the another third side of the another triangular region.

## Patentansprüche

1. Verfahren zum geometrischen Formen eines Abschnitts (10) eines Kopfbandes zum Befestigen einer Patienten-Schnittstellenvorrichtung an dem Kopf eines Patienten, um sich an einen ausgewählten Abschnitt des Kopfes eines vorgesehenen Benutzers anzupassen, wobei das Verfahren Folgendes umfasst:
Bestimmung eines dreieckigen Bereichs (12, 12') auf dem Teil des Kopfbandes, der im Allgemeinen mit dem ausgewählten Teil des Kopfes des vorgesehenen Benutzers übereinstimmt, dem dreieckigen Bereich die durch eine erste Seite (14, 14'), eine zweite Seite (18, 18') und eine dritte Seite (20, 20') definiert ist; und
Bildung einer gekrümmten und schalenförmigen Region (22, 22') in dem Teil der Kopfbedeckung durch Zusammenführen und Koppeln der zweiten und dritten Seite der dreieckigen Region,
**dadurch gekennzeichnet, dass** das Zusammenbringen und Koppeln der zweiten und dritten Seite das Falten des dreieckigen Bereichs entlang einer Faltlinie (26) umfasst, die innerhalb des dreieckigen Bereichs angeordnet ist.

2. Verfahren nach Anspruch 1, wobei die erste Seite an einer Außenkante (16) des Teils der Kopfbedeckung angeordnet ist und die zweite und dritte Seite sich in den Teil der Kopfbedeckung weg von der ersten Seite erstrecken.

3. Verfahren nach Anspruch 1, wobei die erste Seite (14') des dreieckigen Bereichs (12') innerhalb des Teils der Kopfbedeckung angeordnet ist.

4. Verfahren nach Anspruch 3, wobei das Bestimmen eines dreieckigen Bereichs ferner das Bestimmen eines anderen dreieckigen Bereichs (12') neben dem dreieckigen Bereich (12') umfasst; wobei der andere dreieckige Bereich durch eine andere erste Seite (14'), eine andere zweite Seite (18') und eine andere dritte Seite (20') definiert ist, wobei die andere erste Seite mit der ersten Seite zusammenfällt; und wobei das Bilden eines gekrümmten und schalenförmigen Bereichs in dem Abschnitt des Kopfbandes ferner umfasst Zusammenführen und Koppeln der anderen zweiten Seite und der anderen dritten Seite der anderen dreieckigen Region.

## Revendications

1. Procédé de formation géométrique d'une partie (10) d'une garniture de tête pour fixer un dispositif d'interface patient à la tête d'un patient pour se conformer à une partie sélectionnée de la tête d'un utilisateur déterminé, ledit procédé comprenant :
la détermination d'une zone (12, 12') triangulaire sur la partie de la garniture de tête, laquelle coïncide généralement avec la partie sélectionnée de la tête de l'utilisateur déterminé, ladite zone triangulaire étant définie par un premier côté (14, 14'), un deuxième côté (18, 18') et un troisième côté (20, 20') ; et
la formation d'une zone (22, 22') incurvée et bombée dans la partie de la garniture de tête par rapprochement et par couplage des deuxième et troisième côtés de la zone triangulaire,
**caractérisé en ce que** le rapprochement et le couplage des deuxième et troisième côtés comprennent le pliage de la zone triangulaire le long d'une ligne de pliage (26) disposée à l'intérieur de la zone triangulaire.

2. Procédé selon la revendication 1, dans lequel le premier côté est disposé sur un bord extérieur (16) de la partie de la garniture de tête et les deuxième et troisième côtés s'étendent dans la partie de la garniture de tête à partir du premier côté.

3. Procédé selon la revendication 1, dans lequel le premier côté (14') de la zone triangulaire (12') est disposé à l'intérieur de la partie de la garniture de tête.

4. Procédé selon la revendication 3, dans lequel la détermination d'une zone triangulaire comprend en outre la détermination d'une autre zone (12') triangulaire adjacente à ladite zone (12') triangulaire ; dans lequel ladite autre zone triangulaire est définie par un autre premier côté (14'), un autre deuxième côté (18') et un autre troisième côté (20'), ledit autre premier côté coïncidant avec le premier côté ; et dans lequel la formation d'une zone incurvée et bombée dans la partie de la garniture de tête comprend en outre le rapprochement et le couplage de l'autre deuxième côté et de l'autre troisième côté de l'autre zone triangulaire.
